Europäisches Patentamt

European Patent Office

Office européen des brevets

(19)

(11) Publication number: **0 242 977 B1**

# EUROPEAN PATENT SPECIFICATION

(45) Date of publication of patent specification: **06.11.91**     (51) Int. Cl.⁵: **A61K 7/18**

(21) Application number: **87302287.5**

(22) Date of filing: **18.03.87**

(54) **Oral hygiene compositions.**

(30) Priority: **25.03.86 GB 8607333**

(43) Date of publication of application:
**28.10.87 Bulletin 87/44**

(45) Publication of the grant of the patent:
**06.11.91 Bulletin 91/45**

(84) Designated Contracting States:
**CH DE FR GB IT LI NL**

(56) References cited:
**DE-A- 3 526 654         FR-A- 2 406 437
US-A- 2 700 012         US-A- 3 721 691
US-A- 4 057 621         US-A- 4 335 102**

(73) Proprietor: **BEECHAM GROUP PLC
Beecham House Great West Road
Brentford Middlesex TW8 9BD(GB)**

(72) Inventor: **Poile, Steven, c/o Beecham Pro-
ducts Research Dept.
St. George's Avenue
Weybridge, Surrey KT13 ODE(GB)**
Inventor: **Molyneux, Karen, c/oBeecham Pro-
ducts Research Dept
St. George's Avenue
Weybridge, Surrey KT13 ODE(GB)**

(74) Representative: **Lockwood, Barbara Ann et al
Beecham Pharmaceuticals Biosciences Re-
search Centre Great Burgh Yew Tree Bottom
Road
Epsom Surrey KT18 5XQ(GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

**Description**

The present invention relates to oral hygiene compositions, and in particular to dentifrices and mouthwashes having improved anticaries effect.

A variety of anticaries agents have hitherto been used in dentifrices and mouthwashes, including alkali metal fluorides and alkali metal monofluorophosphates. It has now been discovered that enhanced anticaries effect can be achieved when a combination of fluoride ions and oxalate ions is used within a specified weight ratio an pH range.

According to the present invention there is provided an oral hygiene composition comprising up to 7% by weight of the composition of alkali metal oxalate or alkaline earth metal oxalate from 20 to 1500 ppm of fluoride ions, and a dentally acceptable excipient, the composition having a pH of from 4 to 10; and excluding mouthwash compositions comprising solubilised aluminium compounds.

The term alkali metal oxalate and/or alkaline earth metal oxalate as used herein includes mixtures of one or more alkali metal oxalates and/or alkaline earth metal oxalates. Suitable oxalates for use in compositions of the invention may include soluble and sparingly soluble oxalates such as sodium, potassium, lithium, calcium, magnesium, barium and strontium. Particularly preferred oxalates are sodium oxalate, potassium oxalate and calcium oxalate or mixtures thereof.

Preferably the alkali metal oxalate and/or alkaline earth metal oxalate is present in an amount of from 0.0025% to 7% by weight.

The fluoride ions may be provided by an alkali metal fluoride, such as sodium, potassium or lithium fluoride and the use of sodium fluoride is especially preferred. Other suitable fluorides include ammonium, stannous and zinc fluorides.

Alternatively, a monofluorophosphate may be used, preferably an alkali metal monofluorophosphate. Sodium monofluorophosphate, $Na_2PO_3F$, is especially preferred but the corresponding potassium and/or lithium salts can also be employed. The term 'monofluorophosphate' as used herein includes monofluoropolyphosphates, such as those of formulae $Na_4P_3O_9F$; $K_4P_3O_9F$; $Na_3KP_3O_9F$; $(NH_4)_3NaP_3O_9F$ and $Li_4P_3O_9F$.

If desired, a mixture of ionic fluoride and monofluorophosphate may be employed. The total amount of fluoride and monofluorophosphate used is dependent to some extent on the type of composition, but it should be an effective, but non-toxic, amount. Typically the fluoride(s) is present in the oral hygiene composition in an amount to provide a total of from 0.025 to 0.25% of fluorine based on the weight of the oral hygiene composition. The preferred total fluoride level is from 0.05 to 0.15% by weight of the composition.

Preferably, the composition is in the form of a dentifrice, and in this form will usually include a dentally acceptable abrasive. Suitable abrasives are insoluble calcium salts such as calcium carbonate, dicalcium phosphate and calcium pyrophosphate; aluminia, silica and synthetic plastic resin particles.

The silica abrasive can be a precipitated silica or a silica gel, such as the silica xerogels described in US Patent No. 3538230. Preferred silica xerogels are marketed under the trade name 'Syloid' by W.R. Grace and Company, Davison Chemical Division. Preferred precipitated silicas are those marketed under the trade name 'Zeodent' by the J.M. Huber Corporation.

The amount of abrasive will be within conventional limits, and will normally vary from 15% to 50% by weight of the composition.

The compositions of the invention may optionally contain other agents known to enhance the anticaries effect of fluoride and monofluorophosphate, such as calcium glycerophosphate, this being incorporated in a weight ratio of up to 1:13, preferably 1:20 to 1:3, to the total weight of fluoride and/or monofluorophosphate salts.

The composition of the invention may be presented in conventional dentifrice dental cream and dental powder formulations, or as a conventional mouthwash formulation.

The compositions of the invention will also usually contain surfactants, gelling agents and other excipients such as flavouring and colouring agents.

The surfactant is normally a water-soluble non-soap or synthetic organic detergent. Suitable sorfactants include the water-soluble salts of: higher fatty acid monoglyceride monosulphates (for example sodium hydrogenated coconut fatty acid monoglyceride monosulphate); higher alkyl sulphates (for example sodium lauryl sulphate); alkylarysulphonates (for example sodium dodecylbenzenesulphonates); and higher alkyl sulphoacetates (for example sodium lauryl sulphoacetate). There may also be used the saturated higher aliphatic acyl amides of lower aliphatic amino carboxylic acids having 12 to 16 carbon atoms in the acyl radical and in which the amino acid portion is derived from the lower aliphatic saturated mono-amino carboxylic acids having 2 to 6 carbon atoms, such as the fatty acid amides of glycine, sarcosine, alanine, 3-

2

aminopropanoic acid and valine, particularly the N-lauroyl, myristoyl and palmitoyl sarcosinate compounds. Conventional non-ionic surfactants may also be included, if desired.

The surface-active materials are generally present in an amount of 0.05 to 15%, preferably 0.5 to 5% by weight of the composition.

The tooth powders and pastes are prepared in the usual manner. Thus, the ingredients can be mixed in the dry state or as slurries or solutions.

In general the liquid in the dental cream or paste will comprise chiefly water, glycerine, sorbitol and/or a glycol, including suitable mixtures thereof. Suitably, the glycol is propylene glycol or a polyethylene glycol. It is preferred to use also a gelling agent in dental creams such as natural or synthetic gums or gum-like materials, e.g. Irish Moss, gum tragacanth, sodium carboxymethylcellulose, polyvinylpyrrolidone or starch. Irish Moss and sodium carboxymethylcellulose are preferred gelling agents. The gum content is usually up to 10% and preferably 0.01 to 5% by weight of the preparation.

Other materials may be added, such as sweetening agents, for example soluble saccharine, flavouring oils (e.g. oils of spearmint, wintergreen, peppermint), chloroform, colouring or whitening agents (e.g. titanium dioxide), preservative (e.g. sodium benzoate), emulsifying agents silicones, alcohol, menthol, chlorophyllin), antibacterial agents (e.g. chlorhexidine), anti-plaque agents, anti-calculus agents and agents for sensitive dentine (e.g. strontium salts, formaldehyde).

The compositions of the invention may also be in the form of other oral hygiene compositions, for example, the ingredients may be incorporated in mouthwashes of the suspension type, or in compositions which will be chewed by the user, for example, chewing gum, tablets, pastilles and lozenges. These compositions will contain the conventional base materials together with suitable flavours and sweetening agents and may be formulated in known manner.

Oral hygiene compositions of the present invention have been found to inhibit glucose utilisation and lactate production by common oral microorganisms, and this appears to be the mechanism by which the improved anticaries effect of the compositions is achieved.

The compositions for the invention are illustrated by the following examples:

|  | Example I | Example II |
|---|---|---|
|  | % w/w |  |
| Glycerine | 24.00 |  |
| 15% Saccharine solution | 2.00 |  |
| Guar gum | 1.00 | as in Ex I |
| TiO$_2$ | 1.00 |  |
| NaF | 0.22 | 0.11 |
| NaMFP | - | 0.38 |
| Nipagin | 0.05 |  |
| Nipasol | 0.05 |  |
| [1]Sipernat 22S (Thickening silica) | 6.00 |  |
| [2]Tixosil 53 BE (Abrasive Silica) | 14.00 | as in Ex I |
| Flavour | 1.00 |  |
| [3]Adinol C7 (Detergent) | 2.00 |  |
| Sodium Oxalate | 5.00 | 2.5 |
| Water | to 100% |  |

|  | Example III % w/w | Example IV |
|---|---|---|
| Glycerine | 4.00 | 10.00 |
| 70% sorbitol | 16.00 | 10.00 |
| 15% Saccharine solution | 2.00 | 2.00 |
| Na carboxy methyl cellulose | 1.50 | 1.20 |
| Hydroxy ethyl cellulose | 0.10 | - |
| Calcium glycerophosphate | 0.065 | - |
| Polyvinyl pyrrolidone | 0.10 |  |
| Na MFP | 0.38 | 0.76 |
| NaF | 0.11 | 0.10 |
| Na$_2$CO$_3$ | 0.50 | - |
| NaHCO$_3$ | 1.50 | - |
| Sodium lauryl sulphate | 1.88 | 1.60 |
| Hydrated silicone dioxide | 4.00 | - |
| Flavour | 1.00 | 1.00 |
| TiO$_2$ | - | - |
| Sodium oxalate | 2.50 | 3.00 |
| Di calcium phosphate |  | 46.00 |
| Na$_2$HPO$_4$ |  | 1.00 |
| Water | to 100.00 | 100.00 |

[1] Trade mark of Rhone-Poulenc

[2] Trade mark of Degussa

[3] Trade mark of Croda

EP 0 242 977 B1

|  | EXAMPLE V |
|---|---|
|  | % w/w |
| 70% sorbitol | 30.00 |
| 30% saccnarin | 1.40 |
| Na carboxy methyl cellulose | 1.50 |
| Hydroxy ethyl cellulose | 0.10 |
| Polyvinyl pyrrolidine | 0.10 |
| NaMFP | 0.76 |
| CaGP | 0.13 |
| Calcium carbonate | 35.00 |
| Sodium lauryl culphate | 1.88 |
| 10.2% NaOH | 1.00 |
| calcium oxalate | 2.00 |
| Flavour | 1.15 |
| Sipernat 22S | 2.00 |
| Water | to 100.00 |

EXAMPLE VI

A plaque pH study was carried out using mouthwash solutions according to the invention. Sixteen subjects were used each of which abstained from dental hygiene for 24 hours prior to the test and did not eat or drink for one hour before the test. Subjects each rinsed for 1 minute with 10ml of each of the 5 products under test.

Thirty minutes later plaque was sampled from the subjects molar and pre-molar teeth. the sample was mixed with 20u1 of saline and the pH recorded. The subjects then rinsed for 1 minute with 10ml of a 10% w/v sucrose solution and 10 minutes later a further plaque sample was taken from the posterior teeth. The plaque pH was measured as described earlier.

Each subject used each product in a balanced randomised order, the study being double blind in nature.

Results are indicated below

| Test mouthwash | Post challenge |
|---|---|
|  | Plaque pH ± s.d |
| 25 ppm F | 5.68 ± 0.29 |
| 25ppmF + 0.025% Na oxalate | 5.68 ± 0.25 |
| 25 ppm F + 0.05% Na oxalate | 5.74 ± 0.32 |
| 25 ppm F + 0.10% Na oxalate | 5.84 ± 0.31 |
| 0.05% Na oxalate | 5.69 ± 0.24 |

The post challenge pH after rinsing with 25 ppm F and 0.1% Na oxalate was significantly greater than after rinsing with 25 ppm F.

Claims

1. An oral hygiene composition comprising up to 7% by weight of the composition of alkali metal oxalate and/or alkaline earth metal oxalate, from 20 to 1500 ppm of fluoride ions, and a dentally acceptable excipient, the composition having a pH of from 4 to 10; and excluding mouthwash compositions

5

comprising solubilised aluminium compounds.

2. A composition according to claim 1, in which the alkali metal oxalate and/or alkaline earth metal oxalate is present in an amount of from 0.0025% to 7% by weight.

3. A composition according to claim 1 or 2 in which the alkali metal oxalate or alkaline earth metal oxalate comprises sodium oxalate, potassium oxalate or calcium oxalate or mixtures thereof.

4. A composition according to any one of claims 1 to 3, in which the fluoride ions are derived from an alkali metal fluoride and/or an alkali metal monofluorophosphate.

5. A composition according to any one of claims 1 to 4, which is in which the form of a dentifrice.

6. A composition according to any one of claims 1 to 4, which is in which the form of a mouthwash.

**Revendications**

1. Composition d'hygiène orale, caractérisée en ce qu'elle comprend jusqu'à 7 % en poids de la composition d'un oxalate de métal alcalin et/ou d'un oxalate de métal alcalino-terreux, de 20 à 1500 ppm d'ions fluorures et d'un excipient acceptable du point de vue dentaire, la composition ayant un pH de 4 à 10 ; et excluant des compositions d'eau dentifrice comprenant des solutions d'aluminium solubilisées.

2. Composition suivant la revendication 1, caractérisée en ce que l'oxalate de métal alcalin et/ou l'oxalate de métal alcalino-terreux est présent en une quantité de 0,0025 % à 7 % en poids.

3. Composition suivant la revendication 1 ou 2, caractérisée en ce que l'oxalate de métal alcalin ou l'oxalate de métal alcalino-terreux comprend l'oxalate de sodium , l'oxalate de potassium et l'oxalate de calcium ou leurs mélanges.

4. Composition suivant l'une quelconque des revendications 1 à 3, caractérisée en ce que les ions fluorures dérivent d'un fluorure de métal alcalin et/ou d'un monofluorophosphate de métal alcalino-terreux.

5. Composition suivant l'une quelconque des revendications 1 à 4, caractérisée en ce qu'elle est sous forme d'un dentifrice.

6. Composition suivant l'une quelconque des revendications 1 à 4, caractérisée en ce qu'elle est sous forme d'une eau dentifrice.

**Patentansprüche**

1. Mundhygiene-Zusammensetzung, umfassend bis zu 7 Gew.% der Zusammensetzung Alkalimetalloxalat und/oder Erdalkalimetalloxalat, von 20 bis 1500 ppm Fluoridionen und ein für die Zähne verträgliches Exzipients, wobei die Zusammensetzung einen pH-Wert von 4 bis 10 aufweist; und wobei Mundspülungs-zusammensetzungen, die solubilisierte Aluminiumverbindungen enthalten, ausgeschlossen sind.

2. Zusammensetzung nach Anspruch 1, in der das Alkalimetalloxalat und/oder Erdalkalimetalloxalat in einer Menge von 0,0025 bis 7 Gew.% vorhanden ist.

3. Zusammensetzung nach Anspruch 1 oder 2, in der das Alkalimetalloxalat oder Erdalkalimetalloxalat Natriumoxalat, Kaliumoxalat oder Kalziumoxalat oder Gemische davon umfaßt.

4. Zusammensetzung nach einem der Ansprüche 1 bis 3, in der die Fluoridionen von einem Alkalifluorid und/oder einem Alkalimetallmonofluorophosphat abgeleitet sind.

5. Zusammensetzung nach einem der Ansprüche 1 bis 4, die in Form einer Zahnpasta vorliegt.

6. Zusammensetzung nach einem der Ansprüche 1 bis 4, die in Form einer Mundspülung vorliegt.